# EUROPEAN PATENT APPLICATION

(11) **EP 1 925 616 A1**
(43) Date of publication of application: **28.05.2008**
(21) Application number: 06123047.0
(22) Date of filing: 26.10.2006
(51) Int. Cl.: C07D 457/06

(54) **Process for the preparation of crystal forms of cabergoline via stable solvates of cabergoline**

(71) Applicant: LEK Pharmaceuticals d.d., 1526 Ljubljana (SI)
(72) Inventor: The designation of the inventor has not yet been filed
(74) Representative: Liphardt, Bernd

(57) **Abstract**

The present invention relates to a process for producing cabergoline crystal forms. The process comprises preparation of the desired solvate of cabergoline from a solution of cabergoline in chloroaromatic solvents. Afterwards cabergoline crystal form is prepared by recovery from a solvate of cabergoline by drying or with desolvating in a solvent media.

## Description

### FIELD OF THE INVENTION

The present invention relates to the novel solvates of cabergoline and to the process for their conversion into pharmaceutically acceptable crystal forms and to pharmaceutical compositions containing them.

### BACKGROUND OF THE INVENTION

Cabergoline is the generic name of the compound 1 ((6-allylergolin-8β-yl)-carbonyl)-1-(3-dimethylaminopropyl)-3-ethylurea (Formula 1), 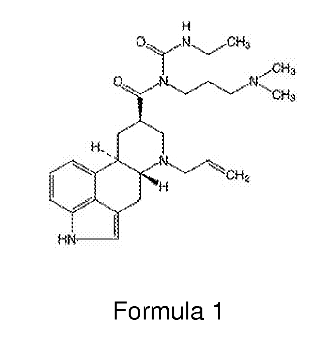 which belongs to the field of pharmaceutical agents for treatment of Parkinson's disease, Restless legs sindrome, treatment of diseases like progressive supranuclear palsy and multisystematic atrophy. The compound was firstly described in the U.S. Patent No. 4,526,892. Eur.J.Med.Chem., 24,421 and GB-2,103,603-B reported the synthesis of cabergoline as well. II Farmaco, 50 (3), 175-178 (1985) described cabergoline crystal form, which was prepared as transparent plates from diethylether and was later designated as crystal form I. Cabergoline was later described in several crystalline forms. The patent application WO 01/70740 describes form V as a toluene solvate and the process of its desolvatation into crystal form I, WO 03/078392 describes form X as another toluene solvate and the process of conversion into form I. WO 01/72747 describes form II and its preparation. WO 01/72746 describes crystal form VII and the process of preparing it. WO 2004/101510 discloses amorphous cabergoline and several new crystalline forms of cabergoline such as form VIII (methyl tert-butyl ether solvate), form XI (xylene solvate), form XII (o-xylene solvate), form XIV (tetrahydropyrane solvate), form XV (cyclohexane solvate), form XVI (p-xylene solvate), form XVI (1,2,4-trimethylbenzene solvate), form XVIII (ethylbenzene solvate) and their preparation. WO 2004/094368 discloses new forms of cabergoline (solvate form A (methyl tert-butyl ether solvate) and an amorphous form, free of crystalline cabergoline.

For pharmaceutical use there is a permanent need for preparation of active pharmaceutical ingredients which are free of impurities and residual solvents and which are stable during technological process for the preparation of a final dosage form. Also pharmaceutical ingredients must stay stable during storage. Above mentioned cabergoline solvates are therefore not acceptable for final dosage forms as they contain solvates. Only acceptable forms of cabergoline are crystal form I, form II and form VII that are unsolvated forms. There is also a need to prepare an intermediate form which could be easily purified and stays stable for a long time. It should be also easily transformed into an unsolvated, pharmaceutically applicable form. We solved that problem by an invention disclosed inhere.

### Summary of the invention

The subject of the invention is a process for the preparation of unsolvated crystal form of cabergoline comprising:
(a) preparation of a solvate of cabergoline from a halogenated aromatic solvent or from a mixture of halogenated aromatic solvents and a second solvent and isolating the solvate of cabergoline,
(b) conversion of a solvate of cabergoline to unsolvated cabergoline crystal form by desolvating of solvate of cabergoline with drying or with desolvating in a solvent media

Another aspect of the invention is a process for a process for the preparation of cabergoline crystal form I comprising:
a) preparation of the solvate of cabergoline from chloroaromatic solvent or from a mixture of solvents to obtain chloroaromatic solvate of cabergoline;
b) conversion of chloroaromatic solvate of cabergoline to cabergoline form I

Another aspect of the invention is a process for the preparation of cabergoline Form VII comprising:
c) preparation of solvate of cabergoline from chloroaromatic solvent to obtain chloroaromatic solvate of cabergoline;
d) conversion of chloroaromatic solvate of cabergoline to cabergoline crystal form VII

Another aspect of the invention is a chlorobenzene solvate of cabergoline.

Another aspect of the invention is a 2-chlorotoluene solvate of cabergoline.

Another aspect of the invention is cabergoline form I used for the preparation of pharmaceutical composition.

Another aspect of the invention is a method for preparing a pharmaceutical composition comprising combining an amount of cabergoline form I and at least one pharmaceutical acceptable carrier.

Another aspect of the invention is a pharmaceutical composition comprising cabergoline form I used for a treatment of Parkinson's disease, restless legs sindrome and treatment of progressive supranuclear palsy and multisystematic atrophy and for treating D2 dopamine receptor associated disorders.

### Description of the figures

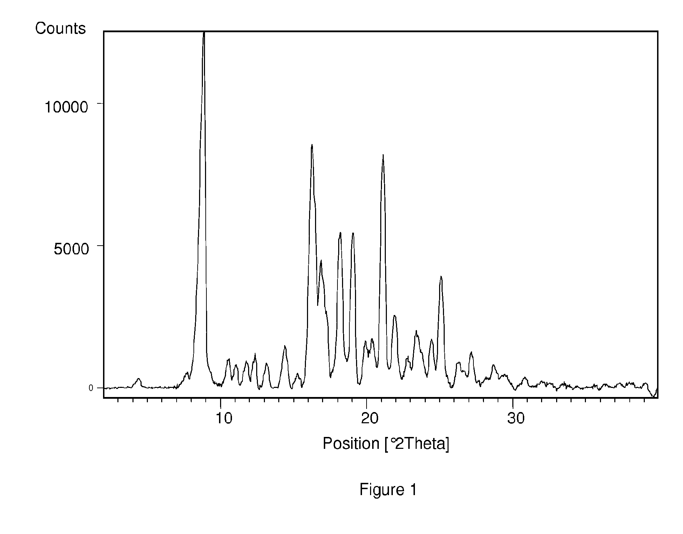

Figure 1: X-ray powder diffraction pattern of chlorobenzene solvate of cabergoline prepared by example 1

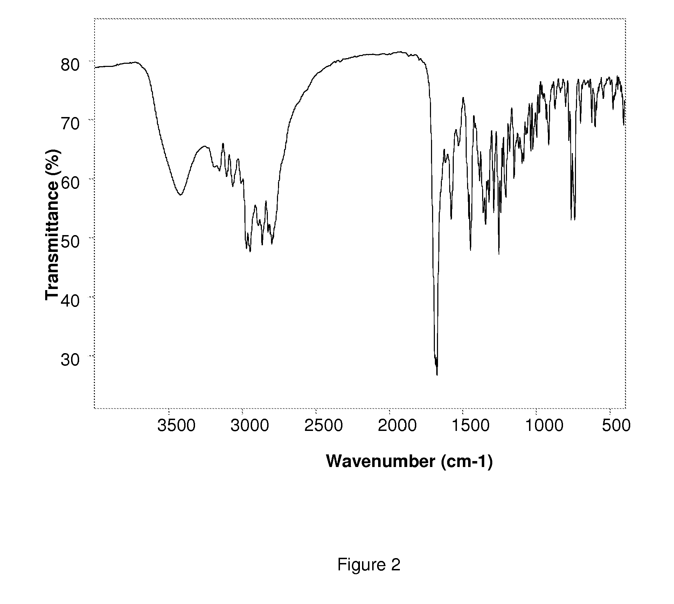

Figure 2: Infrared spectrum of chlorobenzene solvate of cabergoline obtained by experiment prepared by example 1

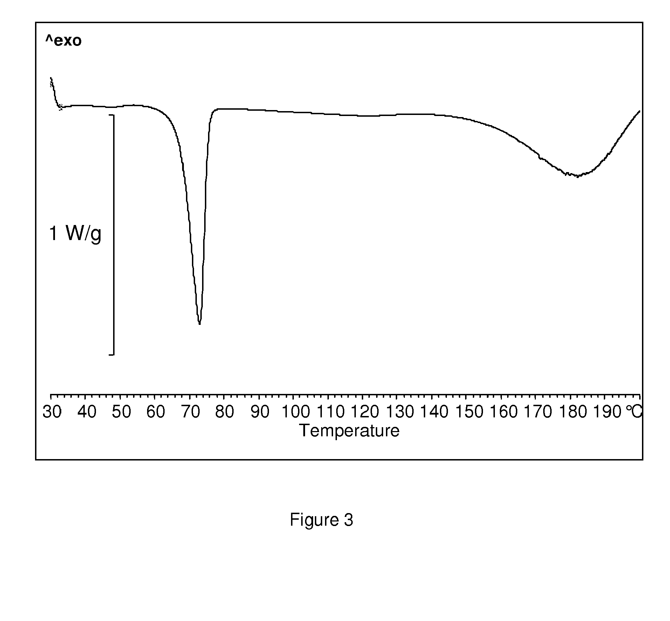

Figure 3: Differential scanning calorimetry pattern of chlorobenzene solvate of cabergoline prepared by example 1

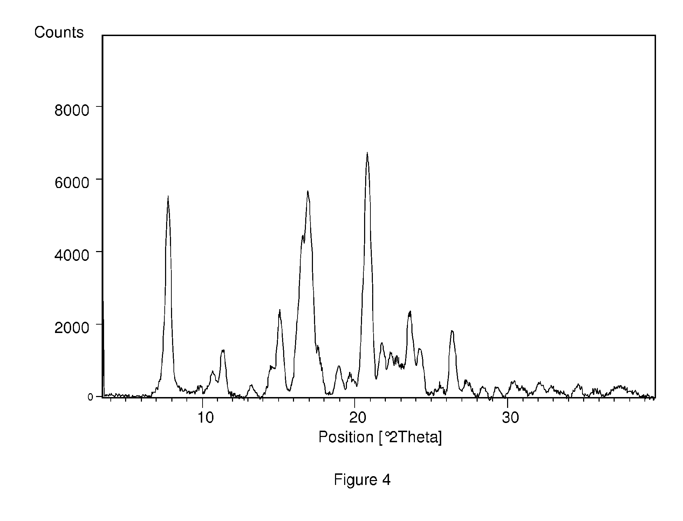

Figure 4: X-ray powder diffraction pattern of 1-chlorotoluene solvate of cabergoline prepared by example 2

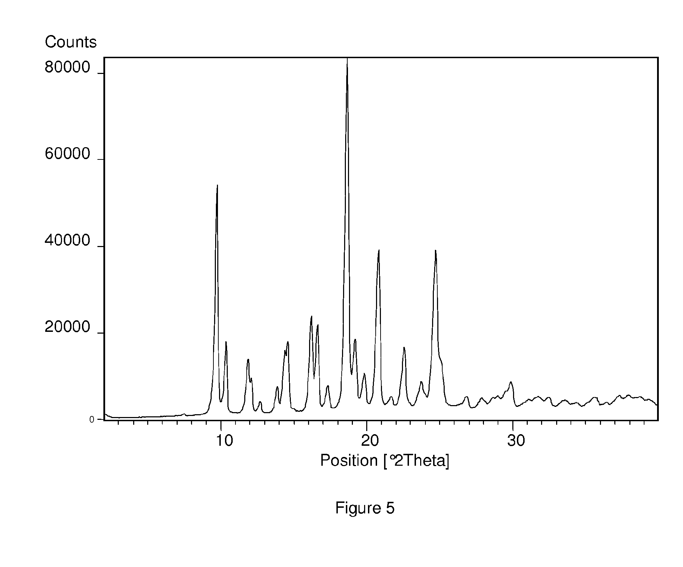

Figure 5: X-ray powder diffraction pattern of cabergoline crystal form I prepared by example 3

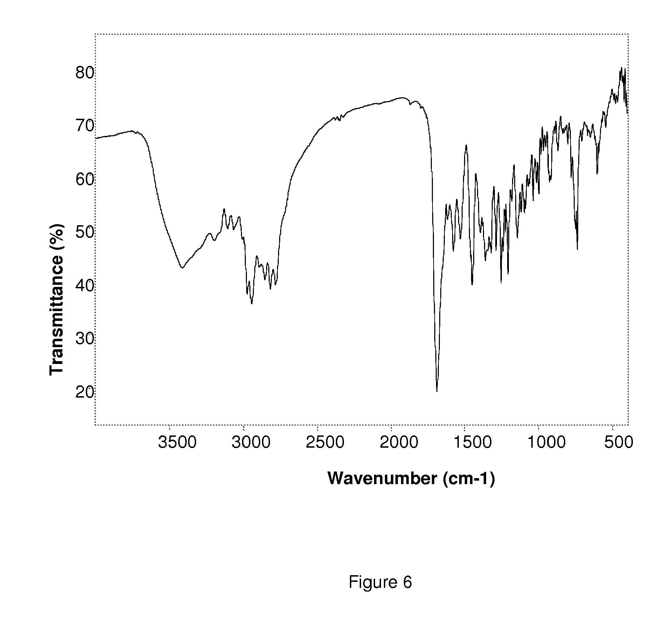

Figure 6: Infrared spectrum of cabergoline form I prepared by example 3

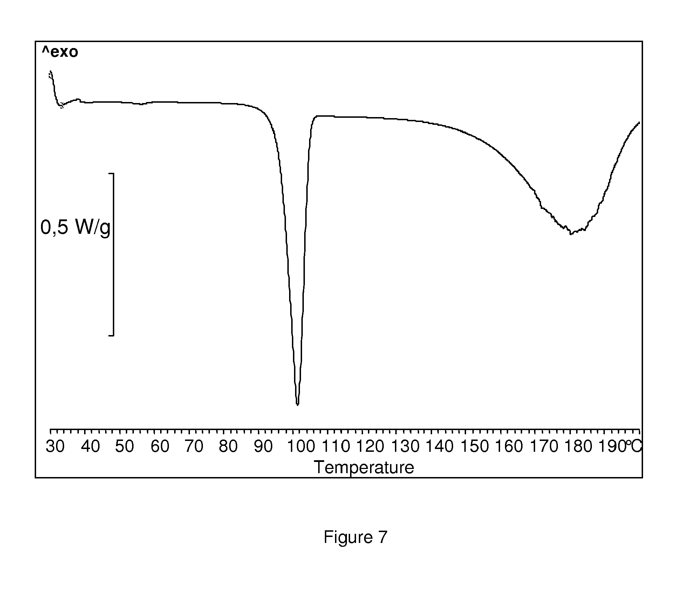

Figure 7: Differential scanning calorimetry pattern of cabergoline form I prepared by example 3

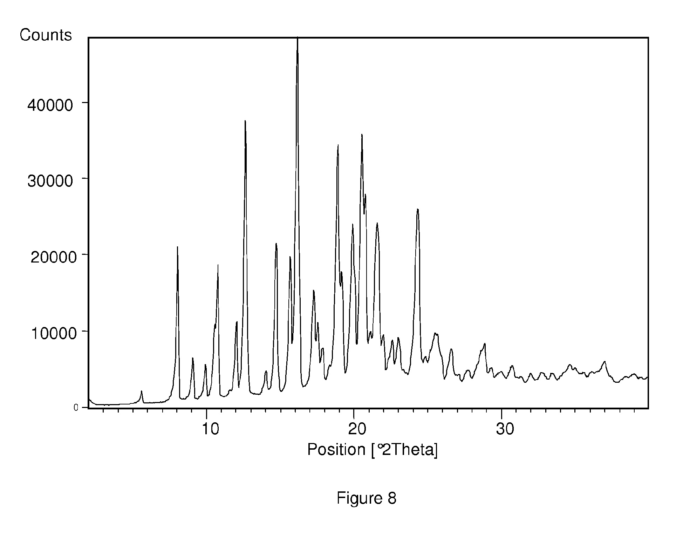

Figure 8: X-ray powder diffraction pattern of cabergoline form VII prepared by example 7.

### Detailed description of the invention

The present invention provides a simple and efficient process for the preparation of new, well defined and for a long term stable solvates of cabergoline with high yields and their conversion into unsolvated forms of cabergoline crystal forms. Crystal forms prepared by the process disclosed in this invention are cabergoline form VII defined in a patent application WO 01/72746 and crystal form I disclosed in II Farmaco, 50 (3), 175-178 (1985) and also in other literature disclosed above. The new solvates of cabergoline are prepared from halogenated aromatic solvents preferably from chloroaromatic solvent. Chloroaromatic solvents used are preferably chloro substituted benzene or chloro substituted toluene. The preferable chloroaromatic solvents therefore are chlorobenzene and 2-chlorotoluene. The chlorobenzene and 2-chlorotoluene solvates are easily prepared with high yield and are transformed into unsolvated cabergoline, preferably into crystal form I and crystal form VII.

Thus, a process for the preparation of unsolvated cabergoline crystal form according to the present invention comprises the steps of:
(a) preparation of a solvate of cabergoline from halogenated aromatic solvents or from a mixture of halogenated aromatic solvent and a second solvent and isolating the solvate of cabergoline and
(b) conversion of a solvate of cabergoline to unsolvated cabergoline crystal form by desolvating with drying or conversion of solvate of cabergoline to cabergoline crystal form with desolvating in alkane or ether solvent media.

### Preparation of a solvate of cabergoline

For the starting cabergoline any known form like crystalline, amorphous, oil or mixture thereof could be used. The process comprises dissolving cabergoline at room temperature in chloroaromatic solvent, selected from a group of chlorobenzene and 2-chlorotoluene or in a mixture of chloroaromatic solvent and a second solvent to obtain a clear solution. An amount of chloroaromatic solvent used is from 2 to 5 ml of chloroaromatic solvent per gram of cabergoline. The second solvent is a solvent selected from a group of hydrocarbons or ethers, preferably from aliphatic hydrocarbons such as heptane.

Afterwards the obtained solution is cool down to -10 to -30 °C, preferably to -15 to - 25 °C, more preferably to -20 °C and let stand for at least 1 hour. During this period a precipitation of a solvate occurs. For the isolation of obtained solvate of cabergoline the mixture is diluted by a third solvent selected from a group of hydrocarbons such as n-heptane, n-heksane, n-pentane, cyclopentane, cyclohexane or methylcyclohexane. Third solvent is added in an amount of 20 ml of third solvent per gram of cabergoline. The mixture is further stirred for 2 to 12 hours, preferably 2 hours at the temperature -10 to -30 °C, preferably to -15 to -25 °C, more preferably to -20 °C. The obtained product is isolated by removing of the solvent by a separation method preferably by filtration and alternatively by decantation or centrifugation. The most preferably used separation method is a filtration through a cold filter under higher pressure. The obtained solid cabergoline substance is identified as solvate of cabergoline with above described chloroaromatic solvent, i.e. selected from a group of chlorobenzene and 2-chlorotoluene.

Cabergoline solvate with halogenated aromatic solvents can be additionally purified by recrystallization from mother solvent, by digestion or by washing with inert solvent such as aliphatic hydrocarbons, preferably by heptane and methylcyclohexane in order to improve chemical purity. For example chlorobenzene solvate can be suspended in heptane for 1-5 hours to yield highly crystalline white powder with total impurity amount bellow 0,3 %.

### Formation of cabergoline crystal form

Solvate of cabergoline can be transformed from solvated form to unsolvated cabergoline crystal form by three following methods.

First method comprises desolvatation carried out with heating of free chloroaromatic solvate of cabergoline at a lower pressure.

Thus, cabergoline crystal form I was prepared by carefully drying of solvate of cabergoline beginning at room temperature (20-25 °C) with very slow gradual rising of temperature up to the desolvatation point. The process is carried out at lower pressure i.e. bellow 100 mbar, preferably at 1 to 10 mbars, preferably at 1 do 5 mbars, more preferably at 1 mbar with prolonged drying for at least for 72 h at 70 °C.

In a case of desolvataiton of 2-chlorotoluene cabergoline solvate it is dried from 20 to 30 °C for 72 hours. The product obtained is cabergoline crystal form I.

In a case of desolvataiton of chlorobenzene cabergoline solvate gradual desolvatation should be carried out below the desolvatation point at 70 °C and temperature should be slowly increased from 55 °C to 70 °C to ensure that chlorobenzene is being removed gradually with simultaneous transformation into form I, preferably drying in high vacuo at 55 °C, 60 °C, 64 °C, 66 °C, 68 °C, 70 °C, each temperature for 24 h. The product obtained is cabergoline crystal form I.

Variation of above process for the preparation of cabergoline crystal form by heating is drying of free chloroaromatic solvate of cabergoline between 40 and 70 °C, more preferably between 50 and 60 °C and most preferably at 55 °C for 24 h to obtain cabergoline crystal form VII.

The second method for a desolvatation comprising desolvatation of chloroaromatic solvate of cabergoline carried out at higher temperatures between 35 and 80 °C, preferably between 40 and 70 °C, more preferably between 50 and 60 °C and most preferably at 55 °C, in a solution of liquid alkane or cycloalkane. Alkane or cycloalkane used are selected from a group of n-heptane, n-hexane, n-pentane, cyclopentane, cyclohexane or methylcyclohexane. An amount of used alkane or cycloalkane is 30 to 50 ml of alkane or cycloalkane per 1 g of solvate of cabergoline prefereably 40 ml of alkane or cycloalkane per 1 g of solvate of cabergoline. The product obtained after desolvatation is identified as cabergoline form I.

The third method comprises desolvatation of a chloroaromatic solvate of cabergoline at around 0 °C in ether solution, preferably in diethyl ether. Therefore chloroaromatic solvate of cabergoline is dissolved in ether and the solution is concentrated to a lower volume followed by the addition of seeds of cabergoline crystal form I. The mixture is then cooled to -25 to -15 °C. Afterwards an alkane preferably n-heptane precooled to -25 to -15 °C is added dropwise while stirring in the time of 1 to 2 hours. The suspension is stirred for next 6 to 7 hours at -25 °C. Finally the suspension was filtered and dried in vacuo at 40 °C. Obtained product is cabergoline crystal form I.

A similar method of preparation of form I can be applied with more soluble forms without concentration of the solvent. Form I can be obtained from concentrated diethylether solution, which is prepared from amorphous cabergoline. Cabergoline from solution is loaded onto seeds of cabegoline form I, below -15 °C to obtain additional quantity of form I. Procedure begins with suspending of cabergoline form I in diethylether at temperatures below -15 °C, preferably below -20 °C, most preferably below -25 °C in amount between 1 g of form I and 30 ml diethylether and 1 g of form I and 2,5 ml of diethylether, preferably in amount between 1 g of form I and 20 ml diethylether and 1 g of form I and 5 ml of diethylether and most preferably in amount of 1 g of form I and 10 ml diethylether. Afterwards amorphous or oily cabergolin is added in amount not exceeded three times as amount of starting cabergoline form I. After entirely amorphous cabergolin is dissolved the liquid alkane or cycloalkane, for example n-heptane, n-hexane, n-pentane, cyclopentane, cyclohexane or methylcyclohexane is added slowly dropwise in the suspension. The amount of alkane should be equal as amount of diethylether. The stirring after complete addition of alkane should continue at least 4 hours, preferably 6 hours, most preferably 12 hours. Further the suspension is filtered and dried in vacuo at 40 °C.)

Cabergoline form I prepared according to this invention can be used for the preparation of pharmaceutical composition. A pharmaceutical composition can be prepared containing cabergoline form I and at least one pharmaceutical acceptable carrier. Pharmaceutical composition comprising cabergoline form I prepared according to this invention can be used for a treatment of Parkinson's disease, restless legs sindrome and treatment of progressive supranuclear palsy and multisystematic atrophy and for treating D2 dopamine receptor associated disorders.

The embodiment of the invention is characterized but not limited by the following experimental examples:

### Example 1: Preparation of chlorobenzene solvate of cabergoline

2,15 g of cabergoline were dissolved in 8 ml of chlorobenzene by agitating at room temperature. The solution was cooled down to -20 °C while stirring. The gel-like material was formed after 15 minutes. Furthermore 40 ml of n-heptane were added and let stirring 1 hour. The obtained fine suspenzion was filtered and washed with 20 ml of cold (-15 °C) n-heptane. The obtained product (2,03 g) was identified as chlorobenzene solvate of cabergoline with 0,34 % of total impurities.

The product can be recrystallized from hot chlorobenzene/heptane mixture to yield cabergoline solvate with 0,13 % of total impurities.

Chlorobenzene solvate of cabergolin is characterized by the following characteristic peaks in XRD diffractogram with values:

| 2θ angle | d | Iᵣₑₗ |
|---|---|---|
| 8.9 | 10.0 | 100 |
| 14.4 | 6.1 | 13 |
| 16.9 | 5.2 | 50 |
| 19.1 | 4.65 | 37 |
| 21.1 | 4.20 | 64 |
| 21.9 | 4.05 | 20 |

The characteristic desolvatation endothermic peak in DSC is detected with onset at 67 °C. The characteristic peak in IR spectrum. appeared at 765 cm⁻¹

### Example 2: Preparation of 2-chlorotoluene solvate of cabergoline

The foamy evaporation containing 1 g of pure cabergoline was dissolved in 2 ml of 2-chlorotoluene. The solution was under stirring cooled to a temperature of -15 °C and subsequently seeded with Form I. After 10 minutes of stirring crystalline slurry was obtained and 10 ml of n-hexane was added into the suspension. The obtained precipitate was filtered and 2-chlorotoluene solvate of cabergoline was obtained which was identified by XRD data.

2-Chlorotoluene solvate of cabergoline is characterized by the following characteristic peaks in XRD diffractogram with values:

| 2θ angle | d | Iᵣₑₗ |
|---|---|---|
| 7.8 | 11.4 | 67 |
| 11.4 | 7.8 | 18 |
| 15.1 | 5.9 | 36 |
| 16.9 | 5.2 | 83 |
| 20.8 | 4.27 | 100 |
| 24.2 | 3.67 | 28 |

### Example 3: Preparation of cabergoline form I with drying

2,03 g of chlorobenzene solvate of cabergoline was placed in the vacuum dryer and dryed according to the following program:
24 hours at 55 °C and 2 mbar
24 hours at 60 °C and 2 mbar
24 hours at 65 °C and 2 mbar
24 hours at 67 °C and 2 mbar
92 hours at 70 °C and 2 mbar

The obtained product (1,87 g) was identified with x-ray diffractometry as cabergoline form I.

### Example 3: Preparation of cabergoline form I with drying

2,08 g of 2-chlorotoluene solvate of cabergoline was placed in the vacuum dryer and dried at 30 °C for one hour. Separated liquid was poured out after while the temperature was carefully raised to 60 °C and dried for 24 h at 1 mbar. The hard mass was grinded to powder which was washed by 5 ml of hexane to yield after filtration 1,86 g of fine powder,.identified with x-ray diffractometry as cabergoline form I.

### Example 4: Preparation of cabergoline form I with desolvatation in n-heptane

1,0 g of chlorobenzene solvate of cabergoline was placed in 40 ml of n-heptane and stirred at 55 °C. Cabergoline solvate was desolvated and converted into a compact substance after couple of minutes. Cabergoline was dried after the decantation of the solvent and identified as cabergoline form I. The yield was above 90 %.

### Example 5: Preparation of cabergoline crystal form I in diethylether

1,0 g of cabergoline form I, prepared by example 4, was placed in the vessel and 6 ml of diethylether precooled to -35 °C were added. Paralelly 1,2 g of cabergoline chlorobenzene solvate from example 2 was dissolved in 20 ml, concentrated to 6 ml, cooled to -15 °C and added to. the suspension of form I. The mixture was stirred at - 15 °C for 1 hour. Afterwards 5 ml of n-heptane precooled to -25 °C were added dropwise while stirring in the time of 1 hour. The enriched suspension was stirred for next 6 hours at -25 °C. Finally the suspension was filtered and dried in vacuo at 40 ºC to obtain 1,8 g of cabergoline form I.

### Example 6: Preparation of cabergoline crystal form I in diethylether

1,0 g of cabergoline form I, prepared by example 4, was placed in the vessel and 10 ml of diethylether precooled to -35 °C were added. The suspension was stirred for 1 hour. Further 1,0 g of amorphous cabergoline from example 1 was added in four portions in the suspension at -25 °C. The suspension was stirred at the same temperature for 1 hour. Afterwards 4 ml of n-heptane precooled to -25 °C were added dropwise while stirring in the time of 1 hour. The enriched suspension was stirred for next 6 hours at -25 °C. Finally the suspension was filtered and dried in vacuo at 40 °C. Obtained product is cabergoline form I.

### Example 7: Preparation of cabergoline crystal form VII with drying

1,0 g of chlorobenzene solvate of cabergoline was heated at 60 °C and 100 kPa 24 hours. The obtained product was characterized as cabergoline form VII.

The products were analysed by following methods:
X-Ray diffraction Method:
Conditions for obtaining powder x-ray diffraction (XRD) patterns: The powder x-ray diffraction patterns were obtained by methods known in the art using Philips X'Pert PRO diffractometer with X'Celerator detector using CuKα radiation (tube operating at 45 kV and 40 mA) in the Bragg-Brentano (reflection) geometry. Data were recorded from 2 to 40 °2θ in steps of 0.033 °2θ and the measurement time of 50 seconds per step. Variable divergence and antiscatter slits were used to maintain 12 mm of sample length irradiated.

### IR spectroscopy Method:

Conditions for obtaining infrared spectra: Fourier transform infrared (FTIR) spectra were recorded with a Nicolet Nexus spectrometer. Spectra over a range of 4000 to 400 cm⁻¹ with a resolution of 2 cm⁻¹ (16 scans) were recorded on KBr tablets.

Differential Scanning calorimetry:
Conditions for obtaining DSC thermograms: Thermograms were obtained with Mettler Toledo DSC822^{e} differential scanning calorimeter. The sample (4-6 mg) was placed in an unsealed aluminium pan with a hole and heated at 5 °C/min in the temperature range from 30 °C to 200 °C.

## Claims

1. A process for the preparation of unsolvated crystal form of cabergoline comprising:
(a) preparation of a solvate of cabergoline from a halogenated aromatic solvent or from a mixture of halogenated aromatic solvents and a second solvent and isolating the solvate of cabergoline,
(b) conversion of a solvate of cabergoline to unsolvated cabergoline crystal form by desolvating of solvate of cabergoline with drying or with desolvating in a solvent media.

2. The process according to claim 1 wherein halogenated aromatic solvent are chloroaromatic solvent.

3. The process according to claim 2 wherein chloroaromatic solvent are selected from a group of chlorobenzene and 2-chlorotoluene.

4. The process according to claim 1 wherein a solvent media in step (b) is selected from a group of alkane and ether.

5. A process for the preparation of cabergoline crystal form I comprising:
(a) preparation of the solvate of cabergoline from chloroaromatic solvent or from a mixture of solvents to obtain chloroaromatic solvate of cabergoline;
(b) conversion of chloroaromatic solvate of cabergoline to cabergoline form l

6. The process according to claim 1 wherein the second solvent is selected form a group of hydrocarbons or ethers.

7. The process according to claim 1 wherein isolating of a solvate of cabergoline comprising cooling a mixture, addition of a third solvent and separation of the product from a solution.

8. The process according to claim 7 wherein cooling means cooling to a temperature of from -10 to -30 °C.

9. The process according to claims 7 and 8 wherein cooling means cooling to a temperature of from -15 to -25 °C.

10. The process according to claim 7 wherein separation method is selected from a group of filtration, decantation and centrifugation.

11. The process according to claim 1 wherein drying in step (b) is performed at the higher temperature.

12. The process according to claim 1 wherein drying in step (b) is performed at the temperature range of from 20 to 30 °C.

13. The process according to claim 1 wherein drying in step (b) is performed at a lower pressure.

14. The process according to claim 1 wherein desolvating in a solvent media is performed in a higher temperature.

15. The process according to claim 14 wherein desolvating in a solvent media is performed in a temperature range between 40 and 70 °C.

16. The process according to claim 1 wherein solvent for solvent media is selected from a group of group of alkane, cycloalkane or ether.

17. The process according to claim 16 wherein alkane is selected from a group of n-heptane, n-hexane, n-pentane.

18. The process according to claim 17 wherein cycloalkane is selected from a group of cyclopentane, cyclohexane or methylcyclohexane.

19. The process according to claim 16 wherein ether is diethylether.

20. The process according to claims 1 and 5 wherein solvate of cabergoline is selected from a group of chlorobenzene solvate of cabergoline and 2-chlorotoluene solvate of cabergoline.

21. The process according to claim 5 wherein an amount of chloroaromatic solvent used is from 2 to 5 ml of chloroaromatic solvent per gram of cabergoline.

22. A process for the preparation of cabergoline Form VII comprising:
(a) preparation of solvate of cabergoline from chloroaromatic solvent to obtain chloroaromatic solvate of cabergoline;
(b) conversion of chloroaromatic solvate of cabergoline to cabergoline crystal form VII

23. The process according to claim 23 wherein the conversion of chloroaromatic solvate of cabergoline at step b) is carried out with drying at the temperatures from 50 to 70 °C.

24. Chlorobenzene solvate of cabergoline.

25. 2-Chlorotoluene solvate of cabergoline.

26. Chlorobenzene solvate of cabergoline which is **characterized by** an X-ray diffraction pattern shown in figure 1.

27. Chlorobenzene solvate of cabergoline which is **characterized by** the following d-values at X-ray diffraction; 10,0 ± 0,1; 6,1 ± 0,1; 5,2 ± 0,1; 4,65 ± 0,01; 4,20 ± 0,01, 4.05 ± 0,01

28. 2-Chlorotoluene solvate of cabergoline which is **characterized by** an X-ray diffraction pattern shown in figure 4.

29. Chlorotoluene solvate of cabergoline which is **characterized by** the following d-values at X-ray diffraction; 11,4 ± 0,1; 7,8 ± 0,1; 5,9 ± 0,1; 5,2 ± 0,1; 4,27 ± 0,01; 3,67 ± 0,01.

30. Cabergoline crystal form I prepared according to claims 1 and 5 used for the preparation of pharmaceutical composition.

31. A method for preparing a pharmaceutical composition comprising cabergoline crystal form I prepared according to a process described in claims 1 and 5 and at least one pharmaceutical acceptable carrier.

32. A pharmaceutical composition comprising cabergoline crystal form I prepared according to claims 1 and 5 used for a treatment of Parkinson's disease, restless legs sindrome and treatment of progressive supranuclear palsy and multisystematic atrophy and for treating D2 dopamine receptor associated disorders
